# EUROPÄISCHE PATENTSCHRIFT

(11) **EP 1 958 660 B1**
(45) Veröffentlichungstag und Bekanntmachung des Hinweises auf die Patenterteilung: **19.08.2009**
(21) Anmeldenummer: 07102503.5
(22) Anmeldetag: 15.02.2007
(51) Int. Cl.: A61M 39/10, F16L 37/107

(54) **Bajonett-Luer-Lockverbindung für eine Insulinpumpe**
Bayonet Luer locking connection for an insulin pump
Raccord Luer Lock à baïonnette pour une pompe à insuline

(43) Veröffentlichungstag der Anmeldung: 20.08.2008
(73) Patentinhaber: F.HOFFMANN-LA ROCHE AG, 4070 Basel (CH); Roche Diagnostics GmbH, 68305 Mannheim (DE)
(72) Erfinder: Aeschlimann, Reto, 3426, Aefligen (CH); Weibel, Michael, 3422, Alchenflüh (CH)
(74) Vertreter: Schwabe - Sandmair - Marx

(56) Entgegenhaltungen:
- EP-A- 0 028 198
- DE-U1-4202004 012 71
- FR-A1- 2 881 503
- US-A- 4 452 473
- US-A- 5 591 143

## Beschreibung

Die vorliegende Erfindung bezieht sich auf eine Verschlusskomponente und einen Verschluss zur Herstellung einer Verbindung, insbesondere einer Fluid-Verbindung, um zum Beispiel eine Pumpe oder Ampulle mit einem Katheter oder Infusions-Set zu verbinden.

Zur Konnektion einer Pumpe oder Ampulle mit einem Katheter oder Infusions-Set wird häufig eine an sich bekannte Luer-Verbindung verwendet, wobei bei einer solchen Luer-Verbindung ein Kraftschluss zwischen einer ersten und einer zweiten Verbindungskomponente, einem so genannten Luer-Male und einem so genannten Luer-Female, hergestellt wird.

Der Luer-Female-Anschluss wird dabei mit einer durch den Anwender erzeugten individuellen Kraft auf den entsprechenden Luer-Male aufgesteckt. Dies kann zu einer undichten Verbindung führen, wenn zu wenig Kraft aufgewandt wird. Drückt der Anwender den Luer-Female jedoch zu fest auf den Luer-Male, kann es sein, dass sich die Verbindung schlecht lösen lässt. Ebenso kann es bei einer mit zu großer Kraft hergestellten Verbindung dazu kommen, dass sich der Luer spaltet, was auch zu einer undichten Stelle führen kann.

Als weitere Konnektionsmöglichkeit ist ein so genannter Luer-Lock bekannt, bei welchem auch ein Formschluss vorliegt. Dabei wird bei einer Luer-Lock-Verbindung zusätzlich zu der bekannten Luer-Verbindung an dem Male-Verbindungsstück noch ein Innengewinde vorgesehen, in welches ein Außengewinde der Female-Verbindung eingreifen kann.

Allgemein sind Luer-Lock-Verbindungen nur für Konnektionen im Bereich von kurzer Zeit ausgelegt. Bei Pumpen ist eine Konnektierung jedoch möglicherweise bis zu einer Woche angebracht und muss über den gesamten Zeitraum für eine sichere und dichte Verbindung sorgen.

Luer-Anschlüsse sind zum Beispiel aus der ISO 594/1 oder der DIN EN 20 594-1 bekannt, deren Lehren bezüglich der Abmessungen und des Aufbaus von Luer-Verbindungen in die vorliegende Anmeldung aufgenommen werden.

Es ist eine Aufgabe der vorliegenden Erfindung eine Verschlusskomponente oder einen Verschluss vorzuschlagen, welche das sichere Herstellen eines lange Zeit haltenden lösbaren Verschlusses ermöglichen.

Diese Aufgabe wird durch die Verschlusskomponente gemäß Anspruch 1 gelöst. Vorteilhafte Ausführungsformen ergeben sich aus den abhängigen Ansprüchen.

Eine erste Verschlusskomponente für einen Verschluss zur Herstellung einer Verbindung, insbesondere einer Fluid-Verbindung, mit einer weiteren zweiten Verschlusskomponente weist einen ersten Adapter oder eine Halterung oder ein Lager auf, an oder in welchem ein verformbares, bewegbares oder verschiebbares Aufsatzelement, also beispielsweise ein Luer-Female-Anschlusselement, gelagert ist. Erfindungsgemäß wirkt zwischen dem ersten Adapter und dem Aufsetzelement eine Kraft, insbesondere eine Federkraft, welche beispielsweise durch ein zwischen dem Adapter und dem Aufsetzelement wirkendes Federelement oder auch durch eine elastische Materialverbindung oder ein Elastomer realisiert werden kann. Hierdurch ist es möglich, dass das Aufsetzelement oder ein Luer-Female mit einer durch das Federelement oder eine Federung vorgegebenen definierten Kraft von dem ersten Adapter weg oder auch innerhalb des Adapters in Richtung auf ein Ansetzelement, wie zum Beispiel einen Luer-Male, gedrückt wird, um somit eine definierte Andruckkraft zwischen dem Aufsetzelement und dem Ansetzelement bzw. dem Luer-Female und dem Luer-Male zu erzeugen, wodurch eine dauerhaft sichere und lösbare Luer-Verbindung hergestellt werden kann, welche unabhängig von der Kraft, mit welcher ein Benutzer die Verschlusskomponente und insbesondere den ersten Adapter aufsetzt, für eine zum Beispiel durch das Federelement vorgegebene definierte Kraft und somit für eine sichere Luer-Verbindung sorgt.

Vorzugsweise ist an dem ersten Adapter mindestens ein Halteelement, wie zum Beispiel eine, zwei oder mehr Rastnasen, welche beispielsweise von dem ersten Adapter radial nach innen oder außen gerichtet vorstehen können, vorgesehen. Mit einem solchen Halteelement oder einer Rastnase kann eine feste Verbindung, wie zum Beispiel eine BajonettVerbindung, mit einem entsprechenden Gegenelement, wie zum Beispiel einem zweiten Adapter, hergestellt werden, um den ersten Adapter, welcher zum Beispiel den Luer-Female trägt, fest mit dem zweiten Adapter, welcher als Pumpen-Adapter ausgebildet sein kann und zum Beispiel nach Einsetzen der Ampulle in eine Pumpe auf die Ampulle oder Pumpe aufgesetzt werden kann, zu verbinden.

Somit kann in einem Bajonett-Luer-Lock-Adapter, welcher zum Beispiel fest an einem Infusionsset befestigt sein kann, der Luer-Female-Anschluss und/oder auch ein Luer-Male-Anschluss durch ein Federelement, oder ein Elastomer in Aufsetzrichtung vorgespannt vorgesehen werden. Wird der Bajonett-Luer-Lock-Adapter auf den Pumpen-Adapter aufgesetzt und mittels einer Bajonettverbindung durch eine Drehung, wie zum Beispiel durch eine 90°-Drehung, mit diesem verrastet, sind die beiden Adapter und damit auch die mit den Adaptern verbundenen Komponenten, also beispielsweise die Pumpe oder Ampulle auf der einen Seite und das Infusionsset auf der anderen Seite, fest miteinander verbunden. Das Aufsetzen des Bajonett-Luer-Lock-Adapters erfolgt gegen die auf den Luer-Female wirkende Federkraft, wobei der Luer-Female durch den Luer-Male nach oben gedrückt wird und somit mit einer durch das Federelement oder ein Elastomer einstellbaren möglich konstanten oder definierten Kraft auf den Luer-Male drückt. Der Bajonett-Luer-Lock-Adapter wird weiterhin durch die Kraft der Feder auch nach oben gedrückt und befindet sich somit ebenfalls in einer definierten Position.

Alernativ oder ergänzend zu der beschreibenen Ausführungsform kann auch oder nur ein elastisches oder Federelement an dem zweiten Adapter, also zum Beispiel zwischen dem Adapter und dem Ansetzelement, vorgesehen sein, wobei das Ansatzelement dann vorzugsweise relativ zu dem zweiten Adapter beweglich oder verschiebbar ist.

Das Luer-Female-Verbindungsstück weist ebenso wie das Luer-Male-Verbindungsstück vorzugsweise eine leicht konisch zulaufende Form auf, wobei der Luer-Male als Kegel oder Kegelstumpf ausgebildet ist und der Luer-Female als Gegenstück dazu eine sich in Einsteckrichtung verjüngende konische Ausformung aufweist. Vorzugsweise beträgt die Steigung der konischen Teilstücke 6%, wobei auf die oben erwähnte Norm, zum Beispiel die ISO 594-1, bezüglich der Ausbildung von Luer-Female- und Luer-Male-Anschlüssen verwiesen wird.

Wird ein 6%-Luer verwendet, d.h. ein Luer-Anschluss, bei welchem die Steigung der Luer-Male-Außenseite 6% beträgt, kann ein Spiel des Luer-Female in axialer Richtung von etwa 2 mm ausreichend sein, um Fertigungstoleranzen der Luer-Verbindung zu kompensieren.

Das Aufsetzelement ist vorzugsweise so in dem ersten Adapter gelagert oder so mit dem ersten Adapter verbunden, dass dieses nicht aus dem ersten Adapter herausfallen oder zum Beispiel durch die Federkraft herausgedrückt werden kann. Beispielsweise kann das Aufsetzelement oder der Luer-Female durch ein oder mehr vorstehende Elemente, wie zum Beispiel radial nach außen weisende Anschlagelemente, Vorsprünge oder Flügel, welche zum Beispiel in Nuten des ersten Adapters geführt werden, vor einem Herausfallen nach unten aus dem ersten Adapter gesichert werden. Ebenso kann auch ein ringförmiges Element an der Außenseite des Aufsetzelementes vorgesehen sein. Diese beispielhaft erwähnten Vorsprünge oder Anschläge des Aufsetzelementes können zum Beispiel beim Einsetzen des Aufsetzelementes in den ersten Adapter an elastischen oder Schnapparmen des ersten Adapters, welche beispielsweise in eine radial nach innen weisende Richtung vorgespannt sind, vorbeigefahren werden und welche nach dem Vorbeifahren des oder der Halteelemente des ersten Adapters wieder in die Ausgangsposition zurückschnappen, um das Aufsetzelement oder Luer-Female vor einem Herausfallen aus dem ersten Adapter zu sichern. Ergänzend oder alternativ kann das Aufsetzelement oder der Luer-Female durch eine obere Aufweitung oder ein konisch zulaufendes Teilstück vor einem Herausfallen nach unten aus dem ersten Adapter gesichert sein. Hierzu kann der erste Adapter zum Beispiel auch eine sich konisch verjüngende Vertiefung oder ein Anschlagelement aufweisen, an welchem zum Beispiel eine Aufweitung des Aufsetzelementes anliegt, wenn das Aufsetzelement in einer maximal zulässigen aus dem ersten Adapter herausgeschobenen oder ausgefahrenen Position ist. Die Herausfallsicherung des Luer-Female kann zum Beispiel auch durch eine Nut in dem Bajonett-Luer-Lock-Adapter oder eine obere Aufweitung des Luer-Female sichergestellt werden, welcher sich in eine trichterförmige obere Öffnung des Bajonett-Luer-Lock-Adapters einfügen kann. Analog kann auch das Ansetzelement in dem zweiten Adapter vor einem Herausfallen gesichert gelagert sein.

Die zweite Verschlusskomponente des Anschlusses oder der Verbindung, welche mit der oben beschriebenen ersten Verschlusskomponente zusammenwirken kann, weist ein Ansetzelement, wie zum Beispiel einen Luer-Male auf, auf welches das oben beschriebene Aufsetzelement, also zum Beispiel der Luer-Female, aufgesetzt werden kann. Des Weiteren ist ein zweiter Adapter vorgesehen, auf welchen der oben beschriebene erste Adapter aufgesetzt werden kann, wobei der zweite Adapter vorzugsweise ein oder mehrere Halteelemente, wie zum Beispiel eine Rastnut aufweist, in welche entsprechende Halteelemente oder Rastnasen des ersten Adapters eingreifen können. Vorteilhaft sind das oder die Halteelemente des zweiten Adapters so ausgebildet, dass der erste Adapter nach dem Aufsetzen auf den zweiten Adapter um zum Beispiel 90° gedreht werden kann, bevor die Verrastung hergestellt wird, um einen Bajonett-Verschluss zwischen dem ersten und dem zweiten Adapter zu realisieren.

Zum Abnehmen des Bajonett-Luer-Lock-Adapters von dem ersten Adapter kann dieser zum Beispiel in Richtung auf den ersten Adapter gedrückt werden, um zum Beispiel den in der Rastnut des zweiten oder Pumpen-Adapters rastenden Bajonettverschlusses gegen die Kraft des Federelementes zu lösen. Anschließend kann der Bajonett-Luer-Lock-Adapter wieder zum Beispiel um 90° zurückgedreht werden und von dem Pumpen-Adapter abgenommen werden.

Allgemein kann für die Erfindung zur Herstellung einer Verbindung mit der oben beschriebenen ersten Verschlusskomponente auch eine Standard-Luer-Ampulle oder ein Standard-Luer-Anschluss verwendet werden.

Die Erfindung wird nachfolgend anhand von Ausführungsbeispielen beschrieben. Es zeigen:
- Figur 1: Eine detaillierte Querschnittsansicht einer ersten Ausführungsform eines Bajonett-Luer-Lock-Adapters; und
- Figur 2: eine perspektivische Ansicht eines auf eine Pumpe aufzusetzenden Bajonett-Luer-Lock-Adapters.

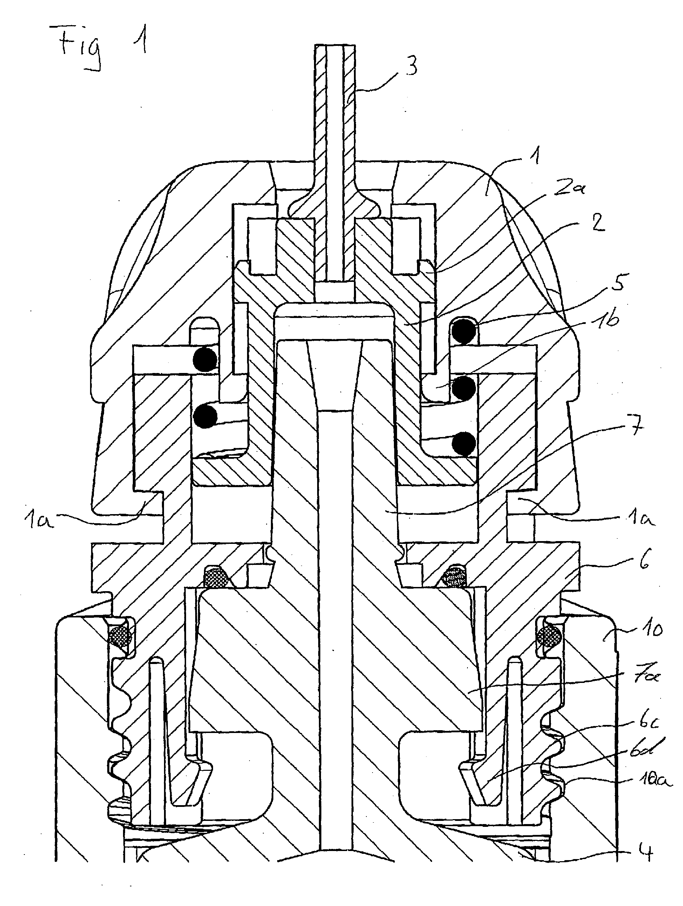

Figur 1 zeigt in einer Querschnittsansicht eine Bajonett-Luer-Lock-Verbindung mit einem ersten Adapter 1, welcher als Bajonett-Luer-Lock-Adapter ausgebildet ist. In dem ersten Adapter 1, welcher etwa rotationssymmetrisch um die Mittelachse der Figur 1 ausgebildet ist, ist als Aufsetzelement ein Luer-Female 2 in axialer Richtung des ersten Adapters 1 verschiebbar vorgesehen. An dem Luer-Female ist ein Katheter oder Schlauch 3 angebracht und vorzugsweise mit dem Luer-Female 2 fest verbunden, durch welchen eine aus der Ampullenpumpe oder Ampulle 4 abzugebende Substanz an ein nicht gezeigtes Infusionsset übertragen wird.

Zwischen dem ersten Adapter 1 und dem Luer-Female 2 ist als elastisches oder federndes Element eine Feder 5 vorgesehen, welche den Luer-Female 2 in der in Figur 1 gezeigten Ausrichtung nach unten von dem ersten Adapter 1 weg drückt.

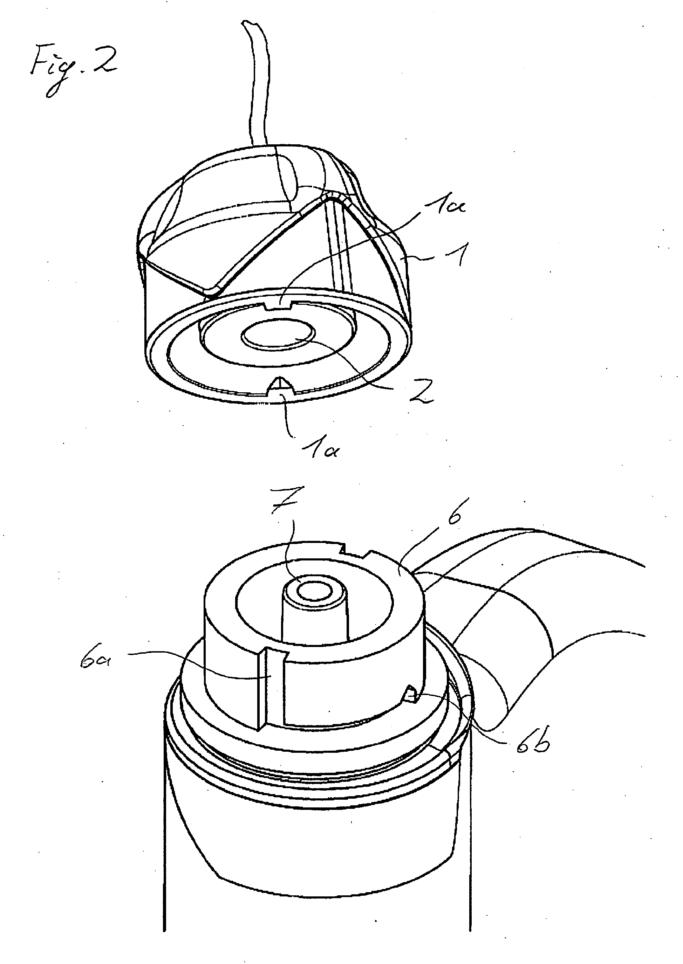

Der erste Adapter 1 ist auf einen zweiten oder Pumpen-Adapter 6 aufgesetzt, welcher an der Ampulle 4 angebracht und zum Beispiel auf die Pumpe 10 aufgeschraubt ist. Der Pumpen-Adapter 6 weist, wie in Figur 2 gezeigt, eine in axialer Richtung des Pumpen-Adapters 6 verlaufende Führungsnut 6a auf, in welcher während des Aufsetzens des ersten Adapters 1 auf den Pumpen-Adapter 6 eine radial nach innen weisende Rastnase 1a des ersten Adapters 1 geführt werden kann. Ist der erste Adapter 1 so weit auf den Pumpen-Adapter 6 aufgeschoben, dass die Rastnase 1a die gesamte Führungsnut 6a in axialer Richtung durchlaufen hat, kann der erste Adapter 1 relativ zum Pumpen-Adapter 6 durch die in Umfangsrichtung verlaufende Führungsnut 6a geführt gedreht werden, wodurch die in der Führungsnut 6a geführte Rastnase 1a des ersten Adapters 1 so weit in Umfangsrichtung des Pumpen-Adapters 6 verschoben wird, bis die Rastnase 1a in die Rastnut 6b des Pumpen-Adapters 6 eingreift. Die Rastnut 6b ist zum Beispiel eine in axialer Richtung auf den ersten Adapter 1 weisende vorgesehene Ausbuchtung der zur Führung der Drehbewegung in Umfangsrichtung vorgesehenen Führungsnut 6a, wobei der erste Adapter 1 in der auf den Pumpen-Adapter 6 aufgesetzten Position durch die Feder 5 von dem Pumpen-Adapter 6 weg gedrückt wird, also in der in Figur 1 gezeigten Ausrichtung nach oben, so dass durch die Rastnut 6b und Rastnase 1a eine Verrastung und somit ein Bajonett-Verschluss zwischen dem ersten Adapter 1 und dem Pumpen-Adapter 6 realisiert werden kann. Die Ampulle 4 ist an ihrem unteren Ende in der Pumpe 10 abgestützt und an ihrem oberen Ende durch den Adapter 6 fixiert und damit gegen axiales Verschieben gesichert.

Ist der erste Adapter 1 auf den Pumpen-Adapter 6 aufgesetzt, so dass die Rastnase 1a in der Rastnut 6b verrastet, liegt der mit dem ersten Adapter 1 über die Feder 5 gekoppelte Luer-Female 2 mit einer durch die Kraft der Feder 5 definierten Andruckskraft auf dem Luer-Male 7 der Ampulle 4 auf.

Erfindungsgemäß wird die Andruckkraft des Luer-Female 2 auf dem Luer-Male 7 somit nicht von einer Andruckkraft eines Benutzers beeinflusst, welcher nur den ersten Adapter 1 auf den Pumpen-Adapter 6 aufsetzt, wodurch die oben erwähnten Probleme einer Luer-Verbindung, welche mit einem zu geringen oder zu großen Druck aufgesetzt wurde, vermieden werden können.

Wie aus Figur 1 ersichtlich, ist der Pumpen-Adapter 6 in eine Pumpe 10 eingeschraubt, in welcher die Ampulle 4 gelagert ist und weist ein Außengewinde 6c auf, welches in ein Innengewinde 10a eines Ampullenaufsatzes eingreift.

Wie aus Figur 1 ersichtlich, weist der Luer-Female 2 radial nach außen vorstehend zwei Vorsprünge oder Flügel oder auch einen umlaufenden Ring 2a auf, welcher eine Herausfallsicherung des Luer-Female 2 aus dem ersten Adapter 1 bildet. Beim Einsetzen des Luer-Female 2 in den ersten Adapter 1 werden die Vorsprünge oder der Ring 2a an den radial nach innen vorgespannten Schnapparmen 1b des Adapters 1 vorbeigeschoben, welche eine Abrundung oder Abschrägung aufweisen, an welchen eine vordere Abrundung oder Abschrägung der Vorsprünge 2a zum Anliegen kommt, wenn der Luer-Female 2 in den ersten Adapter 1 eingesetzt werden soll, wodurch die Schnapparme 1b radial nach außen gedrückt werden. Ist der Luer-Female 2 so weit in den ersten Adapter 1 eingeschoben, dass die Halteelemente 2a an den Schnapparmen 1b vorbeigefahren sind, kehren die radial nach innen vorgespannten Schnapparme 1b wieder in die in Figur 1 gezeigte Ausgangsstellung zurück und können somit ein Herausfallen des Luer-Female 2 aus dem ersten Adapter 1 durch das Halten der Halteelemente 2a verhindern.

Der Pumpen-Adapter 6 weist an seiner der Pumpe 10 zugewandten Unterseite radial nach innen vorgespannte Schnapparme 6d auf, welche hinter einem Vorsprung 7a des Luer-Male 7 eingreifen, so dass die Ampulle 4 im nicht-eingeschraubten Zustand des Adapters 6 in eine Pumpe 10 gegen Herausfallen gesichert ist.

## Patentansprüche

1. Erste Verschlusskomponente für einen Verschluss zur Herstellung einer Verbindung, insbesondere einer Fluidverbindung, mit einem ersten Adapter (1) und einem relativ zu dem ersten Adapter (1) bewegbaren oder verschiebbaren Aufsetzelement (2) und einem elastischen oder federnden Element (5), welches den ersten Adapter (1) mit dem Aufsetzelement (2) koppelt oder zwischen diesen vorgesehen ist.

2. Verschlusskomponente nach Anspruch 1, wobei das Aufsetzelement (2) ein Luer-Female ist.

3. Verschlusskomponente nach einem der vorhergehenden Ansprüche, wobei das Aufsetzelement (2) konisch zulaufend ist.

4. Verschlusskomponente nach einem der vorhergehenden Ansprüche, wobei das Aufsetzelement (2) einen Anschluss für ein Infusions-Set, einen Katheter, eine Kanüle oder einen Schlauch (3) aufweist.

5. Verschlusskomponente nach einem der vorhergehenden Ansprüche, wobei das Aufsetzelement (2) eine Herausfallsicherung oder einen sich konisch aufweitenden oder vorstehenden Bereich aufweist.

6. Verschlusskomponente nach einem der vorhergehenden Ansprüche, wobei der erste Adapter (1) mindestens ein Halteelement oder eine Rastnase (1a) aufweist.

7. Verschlusskomponente nach einem der vorhergehenden Ansprüche, wobei das elastische oder federnde Element eine Feder (5) oder ein Elastomer ist.

8. Zweite Verschlusskomponente mit einem Ansetzelement (7), auf welches das Aufsetzelement (2) der ersten Verschlusskomponente aufgesetzt werden kann und mit einem zweiten Adapter (6), mit welchem der erste Adapter (1) der ersten Verschlusskomponente formschlüssig verbunden werden kann.

9. Zweite Verschlusskomponente nach dem vorhergehenden Anspruch, wobei der zweite Adapter (6) ein Halteelement oder eine Rastnut (6a, 6b) aufweist.

10. Zweite Verschlusskomponente nach dem vorhergehenden Anspruch, wobei das Halteelement (6a, 6b) so ausgelegt ist, dass der erste Adapter (1) in oder an dem zweiten Adapter (6) in axialer Richtung und anschließend in Umfangsrichtung gefiihrt werden kann.

11. Zweite Verschlusskomponente nach einem der drei vorhergehenden Ansprüche mit einem Rastelement oder einer Rastnut (6b) im Bereich der in Umfangsrichtung des zweiten Adapters (6) vorgesehenen Führung (6a).

12. Verschluss mit einer ersten Verschlusskomponente nach einem der Ansprüche 1 bis 7 und einer zweiten Verschlusskomponente nach einem der vier vorhergehenden Ansprüche.

## Claims

1. A first closure component for a closure for establishing a connection, in particular a fluid connection, comprising: a first adaptor (1); a fitting element (2) which can be moved or shifted relative to the first adaptor (1); and an elastic or sprung element (5) which couples the first adaptor (1) to the fitting element (2) or is provided between them.

2. The closure component according to claim 1, wherein the fitting element (2) is a Luer female.

3. The closure component according to any one of the preceding claims, wherein the fitting element (2) is conically tapered.

4. The closure component according to any one of the preceding claims, wherein the fitting element (2) comprises a connector for an infusion set, a catheter, a cannula or a flexible tube (3).

5. The closure component according to any one of the preceding claims, wherein the fitting element (2) comprises a means for securing against falling out or a conically flared or protruding region.

6. The closure component according to any one of the preceding claims, wherein the first adaptor (1) comprises at least one holding element or a latching projection (1a).

7. The closure component according to any one of the preceding claims, wherein the elastic or sprung element is a spring (5) or an elastomer.

8. A second closure component comprising an attaching element (7) onto which the fitting element (2) of the first closure component can be fitted, and comprising a second adaptor (6) to which the first adaptor (1) of the first closure component can be connected in a positive fit.

9. The second closure component according to the preceding claim, wherein the second adaptor (6) comprises a holding element or a latching groove (6a, 6b).

10. The second closure component according to the preceding claim, wherein the holding element (6a, 6b) is designed such that the first adaptor (1) can be guided in or on the second adaptor (6), in the axial direction and then in the circumferential direction.

11. The second closure component according to any one of the preceding three claims, comprising a latching element or a latching groove (6b) in the region of the guide (6a) which is provided in the circumferential direction of the second adaptor (6).

12. A closure comprising a first closure component according to any one of claims 1 to 7 and a second closure component according to any one of the preceding four claims.

## Revendications

1. Premier élément de fermeture d'une fermeture servant à produire une liaison, en particulier une liaison de fluide, avec un premier adaptateur (1) et un élément de réception (2) pouvant être déplacé ou décalé par rapport au premier adaptateur (1) et un élément élastique ou faisant ressort (5) qui est couplé au premier adaptateur (1) avec l'élément de réception (2) ou est prévu entre ceux-ci.

2. Elément de fermeture selon la revendication 1, dans lequel l'élément de réception (2) est un raccord Luer femelle.

3. Elément de fermeture selon l'une des revendications précédentes, dans lequel l'élément de réception (2) est de forme conique.

4. Elément de fermeture selon l'une des revendications précédentes, dans lequel l'élément de réception (2) présente un raccord pour un ensemble de perfusion, un cathéter, une canule ou un tuyau (3).

5. Elément de fermeture selon l'une des revendications précédentes, dans lequel l'élément de réception (2) présente une sécurité anti-déconnexion ou une zone allant en s'élargissant de façon conique ou proéminente.

6. Elément de fermeture selon l'une des revendications précédentes, dans lequel le premier adaptateur (1) présente au moins un élément de retenue ou une encoche d'enclenchement (1a).

7. Elément de fermeture selon l'une des revendications précédentes, dans lequel l'élément élastique ou faisant ressort est un ressort (5) ou un élastomère.

8. Deuxième élément de fermeture comportant un élément d'insertion (7) sur lequel l'élément de réception (2) du premier élément de fermeture peut être placé et comportant un deuxième adaptateur (6) avec lequel le premier adaptateur (1) peut être relié au premier élément de fermeture.

9. Deuxième élément de fermeture selon la revendication précédente, dans lequel le deuxième adaptateur (6) présente un élément de retenue ou une encoche d'enclenchement (6a, 6b).

10. Deuxième élément de retenue selon la revendication précédente, dans lequel l'élément de retenue (6a, 6b) est conçu de telle sorte que le premier adaptateur (1) puisse être conduit dans ou sur le deuxième adaptateur (6) en direction axiale et ensuite, en direction périphérique.

11. Deuxième élément de fermeture selon l'une des trois revendications précédentes, comportant un élément d'arrêt ou une encoche d'enclenchement (6b) dans la zone de guidage (6a) prévue dans la direction périphérique du deuxième adaptateur (6).

12. Fermeture avec un premier élément de fermeture selon l'une des revendications 1 à 7, et un deuxième élément de fermeture selon l'une des quatre revendications précédentes.
